# EUROPEAN PATENT APPLICATION

(11) **EP 1 177 787 A2**
(43) Date of publication of application: **06.02.2002**
(21) Application number: 01306066.0
(22) Date of filing: 13.07.2001
(51) Int. Cl.: A61K 31/00, A61K 31/138, A61K 31/4535, A61K 31/453, A61K 31/404, A61K 31/55, A61K 31/4025, A61K 31/40, A61K 31/4453, A61K 31/439, A61P 27/12

(54) **Use of an estrogen agonist/antagonist for treating cataracts**

(30) Priority: 28.07.2000 US 221441 P
(71) Applicant: Pfizer Products Inc., Groton, CT 06340-5146 (US)
(72) Inventor: Rosati, Robert Louis, Pfizer Global Res. & Devel., Groton, Connecticut 06340 (US)
(74) Representative: Ruddock, Keith Stephen

(57) **Abstract**

This invention relates to methods, pharmaceutical compositions and kits useful in treating cataracts. The compositions are comprised of an estrogen agonist / antagonist and a pharmaceutically acceptable vehicle, carrier or diluent. The compositions and methods of treatment are effective while substantially reducing the concomitant liability of adverse effects associated with estrogen administration.

## Description

### FIELD OF THE INVENTION

This invention relates to compositions, methods and kits for treating cataracts. The compositions, methods and kits utilize estrogen agonist / antagonist compounds.

### BACKGROUND OF THE INVENTION

Epidemiological evidence suggests that estrogens may protect against cataracts. Although women are at higher risk of developing cataracts than are men, this increased risk comes after menopause, when estrogens have waned Livingston, P.M., et al., Dev. Ophthalmol. 26:1-6, (1994); Klein, B.E., et al., Arch. Ophthalmol. 116:219-225, (1998)). In one study of 544 women, early onset of menopause was associated with a 2.9-fold risk of developing cataracts (Shibata, T., et al., Dev. Opthhalmol. 26:25-33, (1994)). Moreover, the results of three small epidemiological studies suggest that postmenopausal estrogen replacement therapy reduces the incidence of cataracts (Klein, B.E., et al., Arch. Ophthalmol. 112:85-91, (1994); Cumming, R.G. and Mitchell, P., Am. J. Epidemiol., 145:242-249, (1997); Benitez del Castillo, J.M., et al., Ophthalmology, 104:970-973, (1997)). An in vivo rat model of age-related cataracts suggests that the protective effect of estrogen is a genomic one (Bigsby, R.M., Proc. Natl. Acad. Sci. USA, 96:9328-9332, (1999)).

In premenopausal women, 17β-estradiol produced by the ovaries is the chief circulating estrogen. Serum estradiol concentrations are low in preadolescent girls and increase at menarche. In women, they range from about 100 pg per milliliter (367 pmol per liter) in the follicular phase to about 600 pg per milliliter (2200 pmol per liter) at the time of ovulation. They may rise to nearly 20,000 pg per milliliter (70,000 pmol per liter) during pregnancy. After menopause, serum estradiol concentrations fall to values similar to or lower than those in men of similar age (5 to 20 pg per milliliter [18 to 74 pmol per liter]) (Yen, S.S.C. and Jaffe, R.B., eds. Reproductive Endocrinology: Physiology, Pathophysiology and Clinical Management, 3rd ed. Philadelphia: W.B. Saunders, (1991)).

Steroidal estrogens are formed ultimately from either androstenedione or testosterone as immediate precursors. The reaction involves aromatization of the A ring, and it is catalyzed in three steps by a monooxygenase enzyme complex (aromatase) that uses NADPH and molecular oxygen as cosubstrates, (Miller, W.L., *Endocr. Rev*., 9:295-318 (1988)). In the first step of the reaction, C 19 (the angular methyl group residing on C 10 of the androgen precursor) is hydroxylated. A second hydroxylation results in the elimination of the newly formed C 19 hydroxymethyl group, and a final hydroxylation on C 2 results in the formation of an unstable intermediate that rearranges to form the phenolic A ring. The entire reaction consumes three molecules of NADPH.

Aromatase activity resides within a transmembrane glycoprotein (P_{450,arom}) that is homologous with the cytochrome P₄₅₀ family of monooxygenases (Nebert, D.W. and Gonzalez, F. J., Annu. Rev. Biochem. 56:945-993, (1987); Corbin, C.J., et al., Proc. Natl. Acad. Sci. USA, 85:8948-8952, (1988)); also essential is a ubiquitous flavoprotein, NADPH-cyctochrome P₄₅₀ reductase. Both proteins are localized in the endoplasmic reticulum of ovarian granulosa cells, testicular Sertoli and Leydig cells, adipocytes, placental synctiotrophoblasts, the preimplantation blastocyst, and various brain regions, including the hypothalamus.

The ovaries are the principle source of estrogen in premenopausal women. The major secretory product is estradiol, synthesized by granulosa cells from androgenic precursors provided by thecal cells. Secreted estradiol is oxidized reversibly to estrone, and both of these estrogens can be converted to estriol. These transformations take place mainly in the liver, where interconversion between estrone and estradiol is catalyzed by 17-hydroxysteroid dehydrogenase.

In men and postmenopausal women, the principle source of estrogen is adipose tissue. In this and in other peripheral tissues, estrone is synthesized from dehydroepiandrosterone, which is secreted by the adrenal cortex. Thus, the contribution of adipose tissue estrogens is regulated, in part by the availability of androgenic precursors (Mendelson, C.R. and Simpson, E.R., Mol. Cell Endocrinol., 52:169-176, (1987)).

Breast cancer is a hormone-dependent disease. Women without functioning ovaries who never receive estrogen replacement do not develop breast cancer. The female-to-male ratio for the disease is about 150 to 1. A host of findings indicate that hormones play a critical role as promoters of the disease. For most epithelial malignancies, a log-log plot of incidence versus age shows a straight-line increase with every year of life. A similar plot for breast cancer shows the same straight line increase, but with a decrease in slope beginning at the age of menopause. The three dates in a woman's life that have a major impact on breast cancer incidence are age of menarche, age at first full-term pregnancy, and age of menopause. Women who experience menarche at age 16 have only 50 to 60 percent of the lifetime breast cancer risk of women who experience menarche at age 12. Similarly, menopause occurring 10 years before the median age (52 years), whether naturally or surgically induced, reduces lifetime breast cancer risk by about 35 percent. Compared with nulliparous women, women who have a first full-term pregnancy by age 18 have 30 to 40 percent the risk of breast cancer. Thus, length of menstrual life--particularly the fraction occurring before the first full-term pregnancy--is a substantial component of the total risk of breast cancer. This factor can account for 70 to 80 percent of the variation in breast cancer frequency in different countries. Therefore, while estrogen replacement therapy is beneficial from a cataract prevention perspective, it can be deleterious from other perspectives such as breast cancer.

International variation has provided some of the most important clues on hormonal carcinogenesis. A woman living to age 80 in North America has 1 chance in 9 of developing invasive breast cancer. Asian women have one-fifth to one-tenth the risk of breast cancer of women in North America or Western Europe. Asian women have substantially lower concentrations of estrogens and progesterone. These differences cannot be explained on a genetic basis, because Asian women living in a Western environment have a risk identical to that of their Western counterparts. These women also differ markedly in height and weight from Asian women in Asia; height and weight are critical regulators of age of menarche and have substantial effects on plasma concentrations of estrogens. (Lippman, M.E., *Breast Cancer*, Chapter 91, in Harrison's Principles of Internal Medicine, 14th ed., 1998).

Menopause occurs naturally at an average age of 50 to 51 years in the USA. As ovaries age, response to pituitary gonadotropins (follicle-stimulating hormone [FSH] and luteinizing hormone [LH]) decreases, initially resulting in shorter follicular phases (thus, shorter menstrual cycles), fewer ovulations, decreased progesterone production, and more irregularity in cycles. Eventually, the follicle fails to respond and does not produce estrogen. The transitional phase, during which a woman passes out of the reproductive stage, begins before menopause. It is termed the climacteric or perimenopause, although many persons refer to it as menopause.

Premature menopause refers to ovarian failure of unknown cause that occurs before age 40. It may be associated with smoking, living at high altitude, or poor nutritional status. Artificial menopause may result from oophorectomy, chemotherapy, radiation of the pelvis, or any process that impairs ovarian blood supply.

### SUMMARY OF THE INVENTION

This invention relates to pharmaceutical compositions useful for the treatment of cataracts. The compositions are comprised of an estrogen agonist / antagonist and a pharmaceutically acceptable carrier, vehicle or diluent.

A second aspect of the invention relates to methods of treating cataracts. Specifically the methods relate to methods of preventing the formation of cataracts or preventing an increase in severity of existing cataracts. The methods comprise the administration of an effective amount of an estrogen agonist / antagonist as described herein.

A third aspect of the invention is that the compositions for and methods of treating cataracts substantially reduce the concomitant liability of adverse effects associated with estrogen administration.

As a fourth aspect, the present invention provides for kits for use by a consumer to treat cataracts. The kits comprise: a) a pharmaceutical composition comprising an estrogen agonist / antagonist and a pharmaceutically acceptable carrier, vehicle or diluent; and, optionally, b) instructions describing a method of using the pharmaceutical compositions for treating cataracts. The instructions may also indicate that the kit is for promoting treating cataracts while substantially reducing the concomitant liability of adverse effects associated with estrogen administration.

As a fifth aspect, the present invention provides for the use of estrogen agonists / antagonists of the present invention for the manufacture of a medicament to treat cataracts. Cataracts are also treated by the medicament while substantially reducing the concomitant liability of adverse effects associated with estrogen administration.

### DETAILED DESCRIPTION OF THE INVENTION

The present invention relates to compositions and methods for treating cataracts. Unless otherwise specified, the following terms have the meanings as defined below.

As used herein, "limit", "treat" and "treatment" are interchangeable terms as are "limiting" and "treating" and, as used herein, include preventative (e.g., prophylactic) and palliative treatment or the act of providing preventative or palliative treatment. The terms include a postponement of development of cataracts or cataract symptoms and/or a reduction in the severity of such symptoms that will or are expected to develop. The terms further include ameliorating existing cataract symptoms, preventing additional symptoms and ameliorating or preventing the underlying metabolic causes of symptoms.

"Cataracts" are defined as a condition that results in loss of luminous clarity within the lens of the eye. Physically, the lens becomes progressively more opaque and, therefore, more resistant to light penetration. This prevents the image in view from properly striking the retina. Cataracts include those that accompany aging which are termed, "degenerative cataracts". Such degenerative cataracts may also be caused by physical insults, such as exposure to ionizing radiation, intense heat, intensely bright lights for long periods ("welder's eye"), and diabetes mellitus. Other forms are caused by diseases, such as rubella, and infectiousinflammatory conditions that affect the eye surface directly by way of the conjunctival tissues.

"Adverse effects associated with estrogen" include breast tenderness, breast cancer, bloating, headache, increased blood clotting and menstrual bleeding in women. Unopposed estrogen therapy increases the risk of endometrial carcinoma. Women on long-term estrogen therapy may have an increased risk that is not reversed by concurrent progestin (N. Engl. J. Med. 332:1589, (1995)). In men, the adverse effects of estrogen include increased blood clotting, gynecomastia, feminization and decreased libido.

A "subject" is an animal including a human that is in need of treatment with the compositions, methods or kits of the present invention. The term "subject" or "subjects" is intended to refer to both the male and female gender unless one gender is specifically indicated.

The term "post-menopausal women" is defined to include not only women of advanced age who have passed through menopause, but also women who have been hysterectomized or for some other reason have suppressed estrogen production, such as those who have undergone long-term administration of corticosteroids, suffer from Cushions' syndrome or have gonadal dysgenesis.

"Breast cancer" is defined as a malignant proliferation of epithelial cells lining the ducts or lobules of the breast.

An "estrogen agonist / antagonist" is a compound that affects some of the same receptors that estrogen does, but not necessarily all, and in some instances, it antagonises or blocks estrogen. It is also known as a "selective estrogen receptor modulator" (SERM). Estrogen agonists / antagonists may also be referred to as antiestrogens although they have some estrogenic activity at some estrogen receptors. Estrogen agonists / antagonists are therefore not what are commonly referred to as "pure antiestrogens". Antiestrogens that can also act as agonists are referred to as Type I antiestrogens. Type I antiestrogens activate the estrogen receptor to bind tightly in the nucleus for a prolonged time but with impaired receptor replenishment (Clark, et al., Steroids 22:707, 1(973); Capony, et al., Mol Cell Endocrinol, 3:233, (1975)).

The methods referred to above for treating cataracts generally refer to benefits and/or survival in the long term. Clinical benefits may be observable within a few weeks, for example 2-3 weeks, however, this does not imply that the patients are not benefiting from the treatment prior to actual clinical observation. It is preferred, however that administration be effected long term; that is for longer than 16 weeks, and preferably longer than 6 months.

Not being bound by any single theory, it is believed that the estrogen agonists / antagonists of the present invention and the compositions containing those estrogen agonists / antagonists treat cataracts through activity at estrogen receptors. The estrogen agonists / antagonists of the present invention exert a positive estrogenic effect in animals in the treatment of catracts. The effects are achieved without the concomitant liability of adverse effects associated with estrogen administration due to the estrogen agonists / antagonists antiestrogen effects in other tissues such as breast tissue.

The estrogen agonists / antagonists of the present invention include the compounds described in U.S. Patent 5,047,431. The structure of these compounds is given by formula (I) below: 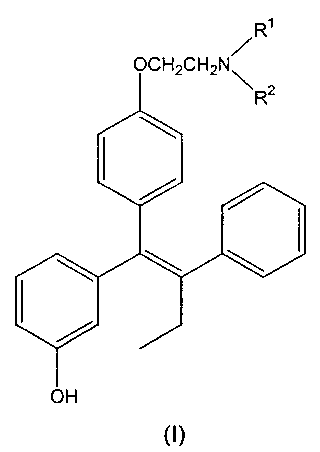 wherein
R¹ and R² may be the same or different and each is a methyl or ethyl group or hydrogen or a benzyl group and optical or geometric isomers thereof; and nontoxic pharmaceutically acceptable salts, N-oxides, esters, quaternary ammonium salts, and prodrugs thereof.

Additional preferred estrogen agonists / antagonists are tamoxifen: (ethanamine,2-[-4-(1,2-diphenyl-1-butenyl)phenoxy]-N,N-dimethyl, (Z)-2-, 2-hydroxy-1,2,3-propanetricarboxylate(1:1)) and other compounds as disclosed in U.S. Patent 4,536,516; 4-hydroxy tamoxifen (i.e., tamoxifen wherein the 2-phenyl moiety has a hydroxy group at the 4 position) and other compounds as disclosed in U.S. Patent 4,623,660; raloxifene: (methanone, [6-hydroxy-2-(4-hydroxyphenyl)benzo[b]thien-3-yl][4-[2-(1-piperidinyl)ethoxy]phenyl]-,hydrochloride) and other compounds as disclosed in U.S. Patents 4,418,068, 5,393,763, 5,457,117, 5,478,847 and 5,641,790; toremifene: (ethanamine, 2-[4-(4-chloro-1,2-diphenyl-1-butenyl)phenoxy]-N,N-dimethyl-, (Z)-, 2-hydroxy-1,2,3-propanetricarboxylate (1:1) and other compounds as disclosed in U.S. Patents 4,696,949 and 4,996,225; centchroman: 1-[2-[[4-(-methoxy-2,2, dimethyl-3-phenylchroman-4-yl)-phenoxy]-ethyl]-pyrrolidine and other compounds as disclosed in U.S. Patent 3,822,287; idoxifene: pyrrolidine, 1-[-[4-[[1-(4-iodophenyl)-2-phenyl-1-butenyl]phenoxy]ethyl] and other compounds as disclosed in U.S. Patent 4,839,155; 6-(4-hydroxy-phenyl)-5-[4-(2-piperidin-1-yl-ethoxy)-benzyl]-naphthalen-2-ol and other compounds as disclosed in U.S. Patent 5,484,795; and {4-[2-(2-azabicyclo[2.2.1]hept-2-yl)-ethoxy]-phenyl}-[6-hydroxy-2-(4-hydroxy-phenyl)-benzo[b]thiophen-3-yl]-methanone and other compounds as disclosed in published international application WO 95/10513. Other preferred compounds include GW 5638 and GW 7604, the synthesis of which compounds is described in Willson et al., J. Med Chem., 1994;37:1550-1552.

Further preferred estrogen agonists / antagonists include EM-652 (as shown in formula (II) and EM-800 (as shown in formula (III)). The synthesis of EM-652 and EM-800 and the activity of various enantiomers is described in Gauthier et al., J. Med. Chem., 1997;40:2117-2122.

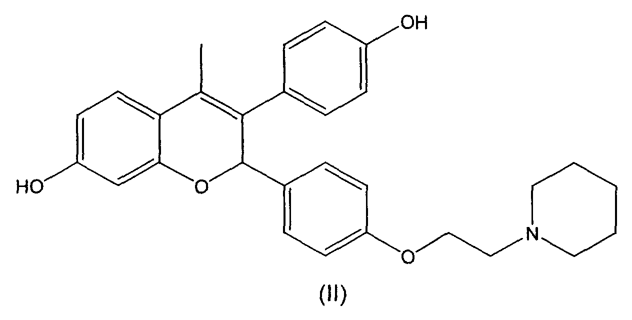

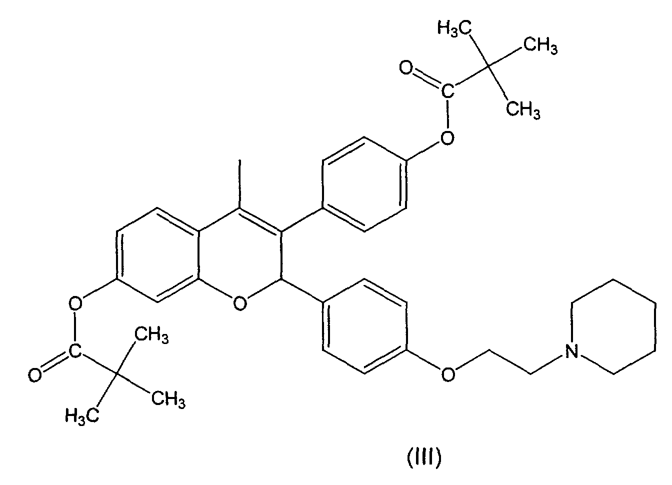

Further preferred estrogen agonists / antagonists include TSE-424 and other compounds disclosed in U.S. Patent 5,998,402, U.S. Patent 5,985,910, U.S. Patent 5,780,497, U.S. Patent 5,880,137, and European Patent Application EP 0802183 A1 including the compounds of the formulas IV or V, below: 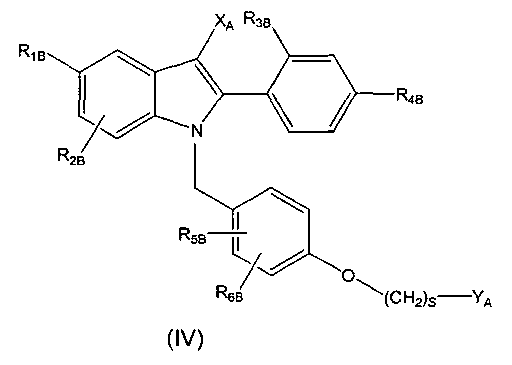 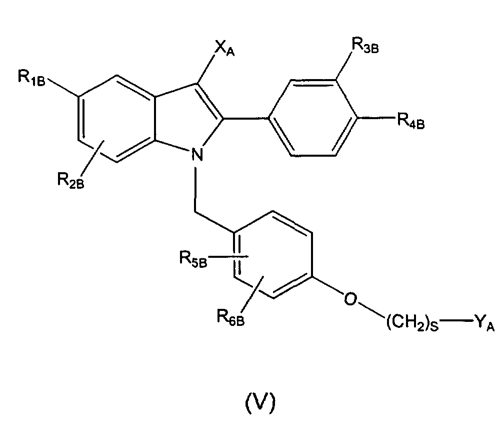 wherein:
R_{1B} is selected from H, OH or the C₁-C₁₂ esters (straight chain or branched) or C₁-C₁₂ (straight chain or branched or cyclic) alkyl ethers thereof, or halogens; or C₁-C₄ halogenated ethers including triflouromethyl ether and trichloromethyl ether,
R_{2B}, R_{3B}, R_{4B}, R_{5B}, and R_{6B} are independently selected from H, OH or the C₁-C₁₂ esters (straight chain or branched) or C₁-C₁₂ alkyl ethers (straight chain or branched or cyclic) thereof, halogens, or C₁-C₄ halogenated ethers including triflouromethyl ether and trichloromethyl ether, cyano, C₁-C₆ alkyl (straight chain or branched), or trifluoromethyl, with the proviso that, when R_{1B} is H, R_{2B} is not OH,
X_{A} is selected from H, C₁-C₆ alkyl, cyano, nitro, triflouromethyl, and halogen;
s is 2 or 3;
Y_{A} is selected from:
   a) the moiety: 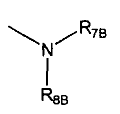 wherein R_{7B} and R_{8B} are independently selected from the group of H, C₁-C₆ alkyl, or phenyl optionally substituted by CN, C₁-C₆ alkyl (straight chain or branched), C₁-C₆ alkoxy (straight chain or branched), halogen, -OH, -CF₃, or -OCF₃;
   b) a five-membered saturated, unsaturated or partially unsaturated heterocycle containing up to two heteroatoms selected from the group consisting of -O-, -NH-, -N(C₁-C₄ alkyl)-, -N=, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, C₁-C₄ alkylamino, di(C₁-C₄)alkylamino, -NHSO₂R_{1B}, -NHCOR_{1B}, -NO₂, and phenyl optionally substituted with 1-3 (C₁-C₄)alkyl;
   c) a six-membered saturated, unsaturated or partially unsaturated heterocycle containing up to two heteroatoms selected from the group consisting of -O-, -NH-, -N(C₁-C₄ alkyl)-, -N=, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, C₁-C₄ alkylamino, di(C₁-C₄)alkylamino, -NHSO₂R_{1B}, -NHCOR_{1B}, -NO₂, and phenyl optionally substituted with 1-3 (C₁-C₄)alkyl;
   d) a seven-membered saturated, unsaturated or partially unsaturated heterocycle containing up to two heteroatoms selected from the group consisting of -O-, -NH-, -N(C₁-C₄ alkyl)-, -N=, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, C₁-C₄ alkylamino, di(C₁-C₄)alkylamino, -NHSO₂R_{1B}, -NHCOR_{1B}, -NO₂, and phenyl optionally substituted with 1-3 (C₁-C₄)alkyl; or
   e) a bicyclic heterocycle containing from 6-12 carbon atoms either bridged or fused and containing up to two heteroatoms selected from the group consisting of -O-, -NH-, -N(C₁-C₄ alkyl)-, -N=, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, C₁-C₄ alkylamino, di(C₁-C₄)alkylamino, -NHSO₂R_{1B}, -NHCOR_{1B}, -NO₂, and phenyl optionally substituted with 1-3 (C₁-C₄) alkyl; and optical or geometric isomers thereof; and nontoxic pharmacologically acceptable acid addition salts, N-oxides, esters, quaternary ammonium salts, and prodrugs thereof.

The more preferred compounds of this invention are those having the general structures IV or V, above, wherein:
R_{1B} is selected from H, OH or the C₁-C₁₂ esters or alkyl ethers thereof, and halogen;
R_{2B}, R_{3B}, R_{4B}, R_{5B}, and R_{6B} are independently selected from H, OH or the C₁-C₁₂ esters or alkyl ethers thereof, halogen, cyano, C₁-C₆ alkyl, or trihalomethyl, preferably trifluoromethyl, with the proviso that, when R_{1B} is H, R_{2B} is not OH;
X_{A} is selected from H, C₁-C₆ alkyl, cyano, nitro, triflouromethyl, and halogen;
Y_{A} is the moiety: 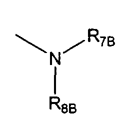
wherein R_{7B} and R_{8B} are selected independently from H, C₁-C₆ alkyl, or combined by -(CH₂)_{w}-, wherein w is an integer of from 2 to 6, so as to form a ring, the ring being optionally substituted by up to three substituents selected from the group of hydrogen, hydroxyl, halo, C₁-C₄ alkyl, trihalomethyl, C₁-C₄ alkoxy, trihalomethoxy, C₁-C₄ alkylthio, C₁-C₄ alkylsulfinyl, C₁-C₄ alkylsulfonyl, hydroxy (C₁-C₄)alkyl, -CO₂H, -CN, -CONH(C₁-C₄), -NH₂, C₁-C₄ alkylamino, C₁-C₄ dialkylamino, -NHSO₂(C₁-C₄), -NHCO(C₁-C₄), and -NO₂; and optical or geometric isomers thereof; and pharmaceutically acceptable salts, N-oxides, esters, quaternary ammonium salts, and prodrugs thereof.

The rings formed by a concatenated R_{7B} and R_{8B}, mentioned above, may include, but are not limited to, aziridine, azetidine, pyrrolidine, piperidine, hexamethyleneamine or heptamethyleneamine rings.

The most preferred compounds of structural formulas IV or V, above, are those wherein R_{1B} is OH; R_{2B} - R_{6B} are as defined above; X_{A} is selected from the group of Cl, NO₂, CN, CF₃, and CH₃; and Y_{A} is the moiety 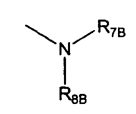 wherein R_{7B} and R_{8B} are concatenated together as -(CH₂)ₜ-, wherein t is an integer of from 4 to 6, to form a ring optionally substituted by up to three subsituents selected from the group of hydrogen, hydroxyl, halo, C₁-C₄ alkyl, trihalomethyl, C₁-C₄ alkoxy, trihalomethoxy, C₁-C₄ alkylthio, C₁-C₄ alkylsulfinyl, C₁-C₄ alkylsulfonyl, hydroxy (C₁-C₄)alkyl, -CO₂H, -CN, -CONH(C₁-C₄)alkyl, -NH₂, C₁-C₄ alkylamino, di(C₁-C₄)alkylamino, -NHSO₂(C₁-C₄)alkyl, -NHCO(C₁-C₄)alkyl, and -NO₂; and optical or geometric isomers thereof; and pharmaceutically acceptable salts, N-oxides, esters, quaternary ammonium salts, and prodrugs thereof.

Another preferred compound is TSE-424 as described by the formula designated herein as formula (Va) below:

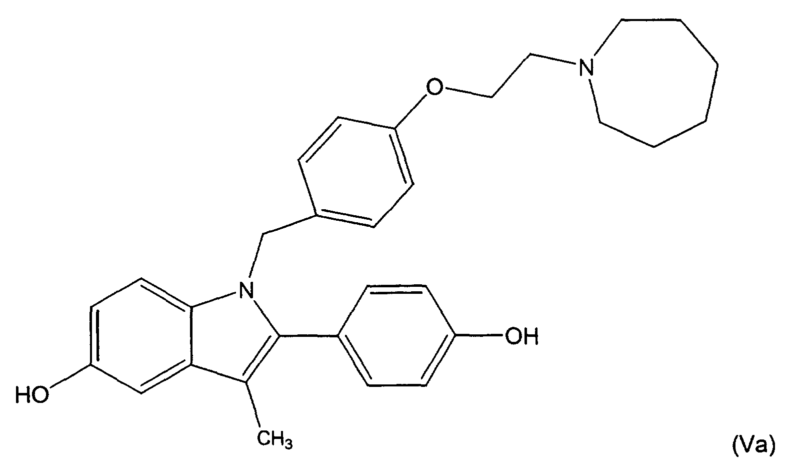

The pharmaceutically acceptable salts of the estrogen agonists / antagonists of this invention may be formed of the compound itself, or of any of its esters, and include the pharmaceutically acceptable salts which are often used in pharmaceutical chemistry. For example, salts may be formed with inorganic or organic acids such as hydrochloric acid, hydrobromic acid, hydroiodic acid, sulfonic acids including such agents as naphthalenesulfonic, methanesulfonic and toluenesulfonic acids, sulfuric acid, nitric acid, phosphoric acid, tartaric acid, pyrosulfuric acid, metaphosphoric acid, succinic acid, formic acid, phthalic acid, lactic acid and the like.

The estrogen agonists / antagonists of this invention, as discussed above, can be administered in the form of acid addition salts. The salts are conveniently formed, as is usual in organic chemistry, by reacting the compound, when basic, with a suitable acid, such as have been described above. The salts are quickly formed in high yields at moderate temperatures, and often are prepared by merely isolating the compound from a suitable acidic wash as the final step of the synthesis. The salt-forming acid is dissolved in an appropriate organic solvent, or aqueous organic solvent, such as an alkanol, ketone or ester. If the estrogen agonist / antagonist of this invention is desired in the free base form, it is isolated from a basic final wash step, according to the usual practice. A preferred technique for preparing hydrochlorides is to dissolve the free base in a suitable solvent and dry the solution thoroughly, as over molecular sieves, before bubbling hydrogen chloride gas through it.

The chemist of ordinary skill will recognize that certain estrogen agonists / antagonists of this invention will contain one or more atoms which may be in a particular stereochemical, tautomeric, or geometric configuration, giving rise to stereoisomers, tautomers and configurational isomers. All such isomers and mixtures thereof are included in this invention. Hydrates and solutes of the estrogen agonists / antagonists of this invention are also included.

The subject invention also includes isotopically-labeled estrogen agonists / antagonists, which are identical to those recited in formulae I, II, III, IV, and V and others but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, sulfur, fluorine and chlorine, such as ²H, ³H, ¹³C, ¹⁴C, ¹⁵N, ¹⁸O, ¹⁷O, ³¹P, ³²P, ³⁵S, ¹⁸F and ³⁶Cl, respectively. Compounds of the present invention, prodrugs thereof, and pharmaceutically acceptable salts of said estrogen agonists / antagonists or of said prodrugs which contain the aforementioned isotopes and/or other isotopes of other atoms are within the scope of this invention. Certain isotopically-labeled estrogen agonists / antagonists of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labeled estrogen agonists / antagonists of formulae I, II, III, IV and V and others of this invention and prodrugs thereof can generally be prepared by carrying out the procedures outlined and/or exemplified in U.S. patent 5,047,431, U.S. Patent 4,536,516, U.S. Patent 4,623,660, U.S. Patent 4,418,068, U.S. Patent 5,393,763, U.S. Patent 5,457,117, U.S. Patent 5,478,847, U.S. Patent 5,641,790, U.S. Patent 4,696,949, U.S. Patent 4,996,225, U.S. Patent 3,822,287, U.S. Patent 4,839,155, U.S. Patent 5,484,795, published international patent application WO 95/10513, Gauthier et al., J. Med. Chem., 1997;40:2117-2122, U.S. Patent 5,998,402, U.S. Patent 5,985,910, U.S. Patent 5,780,497, U.S. Patent 5,880,137, and European Patent Application EP 0802183 and by substituting a readily available isotopically labeled reagent for a non-isotopically labeled reagent.

Pharmaceutical chemists will easily recognize that physiologically active compounds which have accessible hydroxy groups are frequently administered in the form of pharmaceutically acceptable esters. The literature concerning such compounds, such as estradiol, provides a great number of instances of such esters. The estrogen agonists / antagonists of this invention are no exception in this respect, and can be effectively administered as an ester, formed on the hydroxy groups, just as one skilled in pharmaceutical chemistry would expect. It is believed that such esters are metabolically cleaved in the body, yielding the estrogen agonist / antagonist with a free hydroxy group. It is possible, as has long been known in pharmaceutical chemistry, to adjust the rate or duration of action of the compound by appropriate choices of ester groups.

Certain ester groups are preferred as constituents of the estrogen agonists / antagonists of this invention. The estrogen agonists / antagonists of formulae I, II, III, IV and V may contain ester groups at various positions as defined herein above, where these groups are represented as -COOR₉, R₉ is C₁-C₁₄ alkyl, C₁-C₃ chloroalkyl, C₁-C₃ fluoroalkyl, C₅-C₇ cycloalkyl, phenyl, or phenyl mono- or disubstituted with C₁-C₄ alkyl, C₁-C₄ alkoxy, hydroxy, nitro, chloro, fluoro or tri(chloro or fluoro)methyl.

In general, all of the estrogen agonists / antagonists and compositions are prepared according to methods usual in pharmaceutical chemistry and by those procedures outlined and/or exemplified in U.S. Patents 5,047,431, 4,536,516, 4,623,660, 4,418,068, 5,393,763, 5,457,117, 5,478,847, 5,641,790, 4,696,949, 4,996,225, 3,822,287, 4,839,155, 5,484,795, 5,998,402, 5,985,910, 5,780,497, 5,880,137, published international patent application WO 95/10513, published in Gauthier et al., J. Med. Chem., 1997;40:2117-2122, and in Patent and European Patent Application EP 0802183.

Methods of formulation are well known in the art and are disclosed, for example, in Remington: The Science and Practice of Pharmacy, Mack Publishing Company, Easton, Pa., 19th Edition (1995). Pharmaceutical compositions for use within the present invention can be in the form of sterile, non-pyrogenic liquid solutions or suspensions, coated capsules, suppositories, lyophilized powders, transdermal patches or other forms known in the art.

Capsules are prepared by mixing the compound with a suitable diluent and filling the proper amount of the mixture in capsules. The usual diluents include inert powdered substances such as starch of many different kinds, powdered cellulose, especially crystalline and microcrystalline cellulose, sugars such as fructose, mannitol and sucrose, grain flours and similar edible powders.

Tablets are prepared by direct compression, by wet granulation, or by dry granulation. Their formulations usually incorporate diluents, binders, lubricants and disintegrators as well as the compound. Typical diluents include, for example, various types of starch, lactose, mannitol, kaolin, calcium phosphate or sulfate, inorganic salts such as sodium chloride and powdered sugar. Powdered cellulose derivatives are also useful. Typical tablet binders are substances such as starch, gelatin and sugars such as lactose, fructose, glucose and the like. Natural and synthetic gums are also convenient, including acacia, alginates, methylcellulose, polyvinylpyrrolidine and the like. Polyethylene glycol, ethylcellulose and waxes can also serve as binders.

A lubricant may be necessary in a tablet formulation to prevent the tablet and punches from sticking in the die. The lubricant is chosen from such slippery solids as talc, magnesium and calcium stearate, stearic acid and hydrogenated vegetable oils.

Tablet disintegrators are substances which facilitate the disintegration of a tablet to release a compound when the tablet becomes wet. They include starches, clays, celluloses, algins and gums, more particularly, corn and potato starches, methylcellulose, agar, bentonite, wood cellulose, powdered natural sponge, cationexchange resins, alginic acid, guar gum, citrus pulp and carboxymethylcellulose, for example, may be used as well as sodium lauryl sulfate.

Tablets are often coated with sugar as a flavor and sealant, or with film-forming protecting agents to modify the dissolution properties of the tablet. The estrogen agonists / antagonists may also be formulated as chewable tablets, by using large amounts of pleasant-tasting substances such as mannitol in the formulation, as is now well-established in the art.

When it is desired to administer an estrogen agonist / antagonist as a suppository, the typical bases may be used. Cocoa butter is a traditional suppository base, which may be modified by addition of waxes to raise its melting point slightly. Water-miscible suppository bases comprising, particularly, polyethylene glycols of various molecular weights are in wide use.

The effect of the estrogen agonists / antagonists may be delayed or prolonged by proper formulation. For example, a slowly soluble pellet of the estrogen agonist / antagonist may be prepared and incorporated in a tablet or capsule. The technique may be improved by making pellets of several different dissolution rates and filling capsules with a mixture of the pellets. Tablets or capsules may be coated with a film which resists dissolution for a predictable period of time. Even the parenteral preparations may be made long-acting, by dissolving or suspending the estrogen agonist / antagonist in oily or emulsified vehicles which allow it to disperse only slowly in the serum.

The term "prodrug" means compounds that are transformed in vivo to yield an estrogen agonist / antagonist of the present invention. The transformation may occur by various mechanisms, such as through hydrolysis in blood. A good discussion of the use of prodrugs is provided by T. Higuchi and W. Stella, "Prodrugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987.

For example, if an estrogen agonist / antagonist of the present invention contains a carboxylic acid functional group, a prodrug can comprise an ester formed by the replacement of the hydrogen atom of the acid group with a group such as (C₁-C₈)alkyl, (C₂-C₁₂)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-(C₁-C₂)alkylamino(C₂-C₃)alkyl (such as p-dimethylaminoethyl), carbamoyl-(C₁-C₂)alkyl, N,N-di(C₁-C₂)alkylcarbamoyl-(C₁-C₂)alkyl and piperidino-, pyrrolidino- or morpholino(C₂-C₃)alkyl.

Similarly, if an estrogen agonist / antagonist of the present invention comprises an alcohol functional group, a prodrug can be formed by the replacement of the hydrogen atom of the alcohol group with a group such as (C₁-C₆)alkanoyloxymethyl, 1-((C₁-C₆)alkanoyloxy)ethyl, 1-methyl-1-((C₁-C₆)alkanoyloxy)ethyl, (C₁-C₆)alkoxycarbonyloxymethyl, N-(C₁-C₆)alkoxycarbonylaminomethyl, succinoyl, (C₁-C₆)alkanoyl, α-amino(C₁-C₄)alkanoyl, arylacyl and a-aminoacyl, or α-aminoacyl-α-aminoacyl, where each α-aminoacyl group is independently selected from the naturally occurring L-amino acids, P(O)(OH)₂, -P(O)(O(C₁-C₆)alkyl)₂ or glycosyl (the radical resulting from the removal of a hydroxyl group of the hemiacetal form of a carbohydrate).

If an estrogen agonist / antagonist of the present invention comprises an amine functional group, a prodrug can be formed by the replacement of a hydrogen atom in the amine group with a group such as R^{X}-carbonyl, R^{X}O-carbonyl, NR^{X}R^{X'}-carbonyl where R^{X} and R^{X'} are each independently ((C₁-C₁₀)alkyl, (C₃-C₇)cycloalkyl, benzyl, or R^{X}-carbonyl is a natural α-aminoacyl or natural α-aminoacyl-natural α-aminoacyl, -C(OH)C(O)OY^{X} wherein (Y^{X} is H, (C₁-C₆)alkyl or benzyl), -C(OY^{X0})Y^{X1} wherein Y^{X0} is (C₁-C₄) alkyl and Y^{X1} is ((C₁-C₆)alkyl, carboxy(C₁-C₆)alkyl, amino(C₁-C₄)alkyl or mono-N- or di-N,N-(C₁-C₆)alkylaminoalkyl, -C(Y^{X2})Y^{X3} wherein Y^{X2} is H or methyl and Y^{X3} is mono-N- or di-N,N-(C₁-C₆)alkylamino, morpholino, piperidin-1-yl or pyrrolidin-1-yl.

The methods of treating cataracts involve the administration of an effective amount of an estrogen agonist / antagonist. More specifically, the methods include methods of preventing the formation of cataracts and preventing an increase in severity of existing cataracts. The treatment of cataracts with the estrogen agonists / antagonists of the invention substantially reduces the concomitant liability of adverse effects associated with estrogen administration.

As used herein, the term "effective amount" means an amount of estrogen agonist / antagonist of the methods of the present invention that is capable of treating the symptoms of the described pathological conditions. The specific dose of an estrogen agonist / antagonist administered according to this invention will, of course, be determined by the particular circumstances surrounding the case including, for example, the estrogen agonist / antagonist administered, the route of administration, the state of being of the patient, and the severity of the condition being treated.

The dose of an estrogen agonist / antagonist of this invention to be administered to a human is rather widely variable and subject to the judgement of the attending physician. It should be noted that it may be necessary to adjust the dose of a compound when it is administered in the form of a salt, such as a laureate, the salt forming moiety of which has an appreciable molecular weight. The general range of effective administration rates of the estrogen agonists / antagonists is from about 0.01 mg/day to about 500 mg/day. A preferred rate range is from about 0.05 mg/day to 250 mg/day. Of course, it is often practical to administer the daily dose of estrogen agonist / antagonist in portions, at various hours of the day. However, in any given case, the amount of estrogen agonist / antagonist administered will depend on such factors as the solubility of the active component, the formulation used and the route of administration.

The route of administration of the estrogen agonists / antagonists of this invention is not critical. The estrogen agonists / antagonists are known to be absorbed from the alimentary tract, and so it is usually preferred to administer an estrogen agonist / antagonist orally for reasons of convenience. However, the estrogen agonists / antagonists may equally effectively be administered percutaneously, or as suppositories for absorption by the rectum, if desired in a given instance. All of the usual types of compositions may be used, including tablets, chewable tablets, capsules, solutions, parenteral solutions, troches, suppositories and suspensions. Compositions are formulated to contain a daily dose, or a convenient fraction of daily dose, in a dosage unit, which, for example, may be a single tablet or capsule or convenient volume of a liquid.

Advantageously, the present invention also provides kits for use by a consumer for treating cataracts. The kits comprise a) a pharmaceutical composition comprising an estrogen agonist / antagonist and a pharmaceutically acceptable carrier, vehicle or diluent; and, optionally, b) instructions describing a method of using the pharmaceutical composition for treating cataracts. The instructions may also indicate that the kit is for treating cataracts while substantially reducing the concomitant liability of adverse effects associated with estrogen administration.

A "kit" as used in the instant application includes a container for containing the composition. The container can be in any conventional shape or form as known in the art which is made of a pharmaceutically acceptable material, for example a paper or cardboard box, a glass or plastic bottle or jar, a re-sealable bag (for example, to hold a "refill" of tablets for placement into a different container), or a blister pack with individual doses for pressing out of the pack according to a therapeutic schedule. The container employed can depend on the exact dosage form involved, for example a conventional cardboard box would not generally be used to hold a liquid suspension. It is feasible that more than one container can be used together in a single package to provide a single dosage form. For example, tablets may be contained in a bottle which is in turn contained within a box.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process, recesses are formed in the plastic foil. The recesses have the size and shape of individual tablets or capsules to be packed or may have the size and shape to accommodate multiple tablets and/or capsules to be packed. Next, the tablets or capsules are placed in the recesses accordingly and the sheet of relatively stiff material is sealed against the plastic foil at the face of the foil which is opposite from the direction in which the recesses were formed. As a result, the tablets or capsules are individually sealed or collectively sealed, as desired, in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It is desirable to provide a written memory aid, where the written memory aid is of the type containing information and/or instructions for the physician, pharmacist or other health care provider, or patient, e.g., in the form of numbers next to the tablets or capsules whereby the numbers correspond with the days of the regimen which the tablets or capsules so specified should be ingested or a card which contains the same type of information. Another example of such a memory aid is a calendar printed on the card e.g., as follows "First Week, Monday, Tuesday," ... etc .... "Second Week, Monday, Tuesday, ..." etc. Other variations of memory aids will be readily apparent. A "daily dose" can be a single tablet or capsule or several tablets or capsules to be taken on a given day.

Another specific embodiment of a kit is a dispenser designed to dispense the daily doses one at a time in the order of their intended use. Preferably, the dispenser is equipped with a memory-aid, so as to further facilitate compliance with the regimen. An example of such a memory-aid is a mechanical counter which indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

Based on a reading of the present description and claims, certain modifications to the compositions, methods and kits described herein will be apparent to one of ordinary skill in the art. The claims appended hereto are intended to encompass these modifications.

All references and patents cited herein are incorporated by reference.

### EXAMPLES

### Example 1: Estrogen Receptor Binding.

Estrogen and estrogen agonist / antagonist binding affinity is measured by the following protocol:
cDNA cloning of human ERα: The coding region of human ERα is cloned by RT-PCR from human breast cancer cell mRNA using Expand™ High Fidelity PCR System according to manufacturer's instructions (Boehringer-Mannheim, Indianapolis, IN). PCR products are cloned into pCR2.1 TA Cloning Kit (Invitrogen, Carlsbad, CA) and sequenced. Each receptor coding region is subcloned into the mammalian expression vector pcDNA3 ((Invitrogen, Carlsbad, CA).

Mammalian cell expression. Receptor proteins are overexpressed in 293T cells. These cells, derived from HEK293 cells (ATCC, Manassas, VA), have been engineered to stably express large T antigen and can therefore replicate plasmids containing a SV40 origin of replication to high copy numbers. 293T cells are transfected with either hERα-pcDNA3 or hERβ-pcDNA3 using lipofectamine as described by the manufacturer (Gibco/BRL, Bethesda, MD). Cells are harvested in phosphate buffered saline (PBS) with 0.5 mM EDTA at 48 h post-transfection. Cell pellets are washed once with PBS/EDTA. Whole cell lysates are prepared by homogenization in TEG buffer (50 mM Tris pH 7.4, 1.5 mM EDTA, 50 mM NaCI, 10% glycerol, 5 mM DTT, 5 µg/ml aprotinin, 10 µg/ml leupeptin, 0.1 mg/ml Pefabloc) using a dounce homogenizer. Extracts are centrifuged at 100,000 x g for 2 h at 4°C and supernatants are collected. Total protein concentrations are determined using BioRad reagent (BioRad, Hercules, CA).

Competition binding assay. The ability of estrogen agonists / antagonists to inhibit [³H]-estradiol binding is measured by a competition binding assay using dextran-coated charcoal as has been described (Leake RE, Habib F 1987 Steroid hormone receptors: assay and characterization. In: B. Green and R.E. Leake (eds). Steroid Hormones a Practical Approach. IRL Press Ltd, Oxford. 67-92.) 293T cell extracts expressing either hERα or hERβ are incubated in the presence of increasing concentrations of estrogen agonist / antagonist and a fixed concentration of [³H]-estradiol (141 µCi/mmol, New England Nuclear, Boston, MA) in 50 mM TrisHCl pH 7.4, 1.5 mM EDTA, 50 mM NaCl, 10% glycerol, 5 mM DTT, 0.5 mg/mL β-lactoglobulin in a final volume of 0.2 mL. All estrogen agonists / antagonists are dissolved in dimethylsulfoxide. The final concentration of receptor is 50 pM with 0.5 nM [³H]-estradiol. After 16 h at 4°C, dextran-coated charcoal (20 µL) is added. After 15 min at room temperature the charcoal is removed by centrifugation and the radioactive ligand present in the supernatant is measured by scintillation counting. All reagents are obtained from Sigma (St. Louis, MO) unless otherwise indicated.

### Example 2: Inhibition of In Vitro Human Breast Tumor Cell Growth.

The *in vitro* antiproliferative effects of estrogen agonists / antagonists are tested using two types of human breast cancer cell lines: first, MCF-7 cells, which contain ER as well as progesterone receptors (PgR), and second, MDA-MB-231 cells, which lack ER and PgR, and enable the determination of an effect that is independent of the ER mechanism. The effect of estrogen agonists / antagonists on the growth of these different cell lines is determined by incubation of the cells with various estrogen agonist / antagonist concentrations for 6 days. The antiproliferative effects are then determined by direct cell counts.

### Example 3 : Inhibition of Cataract Formation.

The effects of the estrogen agonists / antagonists of the present invention are assessed on female Sprague-Dawley rats. At an age of 45 to 60 days, rats are ovariectomized. Each animal receives a single intravenous injection of 50 mg/kg methylnitrosourea (MNU (dissolved in phosphate-buffered saline (PBS) and injected through the tail vein within 15 minutes of preparation) and a treatment Silasitic™ capsule containing estrogen agonist / antagonist is placed subcutaneously on the back. A placebo group receives an empty Silastic capsule. Non-ovariectomized rats are also injected with MNU and serve as the normal animal control.

The eyes of each animal are examined daily for gross changes and abnormalities. At 40 weeks post MNU injection, the animals are euthanized. Entire eyes are removed from the euthanized animals, slit open across the cornea and immersed in fixative (neutral formalin/ethanol/acetic acid/water 2:3:1:3) for 2 weeks as described by Roy et al., Hiroshima J. Med. Sci., 1989;38:95-98. The eyes are then processed and embedded in paraffin. Six-micrometer sections are prepared and stained with hematoxylin and eosin for examination of lens histology.

The eyes of euthanized estrogen agonist / antagonist treated animals and euthanized ovariectomized placebo control animals are extruded and slit open around the cornea and the lenses carefully removed. The lens from each eye is placed in a shallow culture dish containing PBS. The dish is placed on the stage of a dissecting microscope with its zoom objective lens set at 1.5X; a charge-coupled device color video camera (model DXC-960MD, Sony) is attached to one ocular. The lens is viewed with transmitted light and the image captured using an imaging program (IPLAB SPECTRUM, Signal Analytics, Vienna, VA) run on a computer. A 2-mm thick piece (1 cm square) of opaque, white Teflon™ is included in the microscope field for measurement of transmitted light. The intensity of transmitted light (in arbitrary units) transmitted at the center of the lens is measured by the imaging program. Likewise, the intensity of light transmitted through the culture dish to a position outside the lens is measured to define 100% transmission. The units of light measured from the Teflon piece are considered background and are used to correct the light transmission measurements made at the lens and outside the lens. The light passing through the lens is calculated as the percentage transmission.

## Claims

1. The use of an estrogen agonist / antagonist for the manufacture of a medicament for the treatment of cataracts.

2. A use as claimed in claim 1 wherein said estrogen agonist / antagonist is a compound of formula (I): 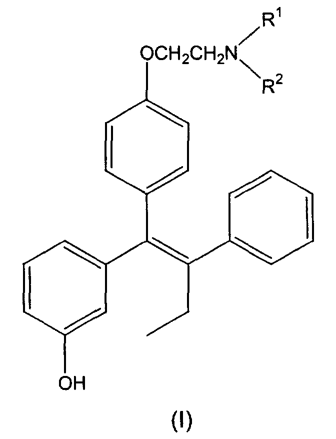 wherein
R¹ and R² may be the same or different and each is a methyl or ethyl group, or hydrogen or a benzyl group; or an optical or geometric isomer thereof; or a pharmaceutically acceptable salt, N-oxide, ester, quaternary ammonium salt or prodrug thereof.

3. A use as claimed in claim 1 wherein said estrogen agonist / antagonist is selected from the group consisting of tamoxifen, 4-hydroxy tamoxifen, raloxifene, toremifene, centchroman, idoxifene, 6-(4-hydroxy-phenyl)-5-[4-(2-piperidin-1-yl-ethoxy)-benzyl]-naphthalen-2-ol, {4-[2-(2-aza-bicyclo[2.2.1]hept-2-yl)-ethoxy]-phenyl}-[6-hydroxy-2-(4-hydroxy-phenyl)-benzo[b]thiophen-3-yl]-methanone, EM-652, EM-800, TSE-424, GW 5638, GW 7604 and optical or geometric isomers thereof; and pharmaceutically acceptable salts, N-oxides, esters, quaternary ammonium salts, and prodrugs thereof.

4. A use as claimed in claim 1 wherein said estrogen agonist / antagonist is a compound of formula (II): 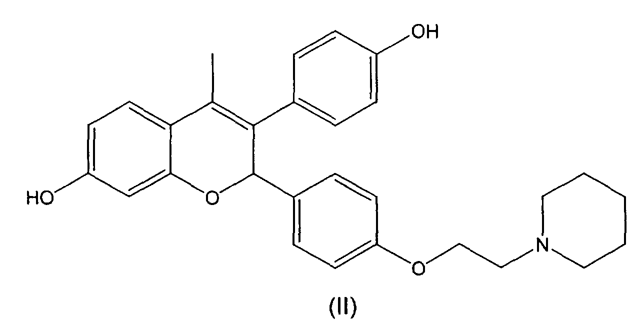 or an optical or geometric isomer thereof; or a pharmaceutically acceptable salt, N-oxide, ester, quaternary ammonium salt or prodrug thereof.

5. A use as claimed in claim 1 wherein said estrogen agonist / antagonist is a compound of formula (III): 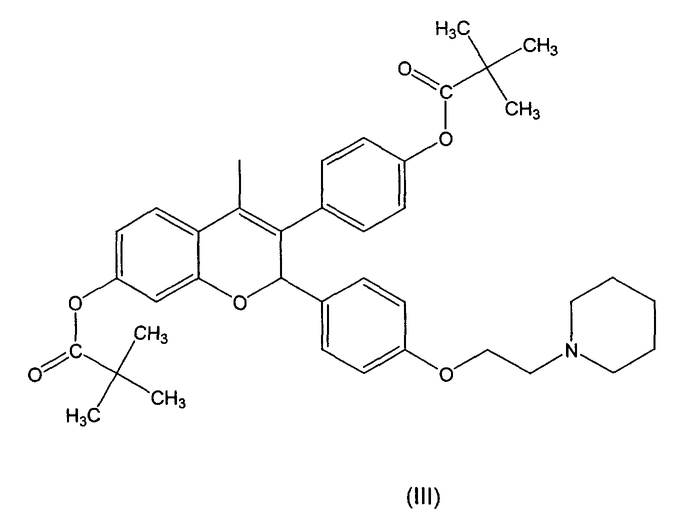 or an optical or geometric isomer thereof; or a pharmaceutically acceptable salt, N-oxide, ester, quaternary ammonium salt or prodrug thereof.

6. A use as claimed in claim 1 wherein said estrogen agonist / antagonist is a compound selected from the formulas IV or V: 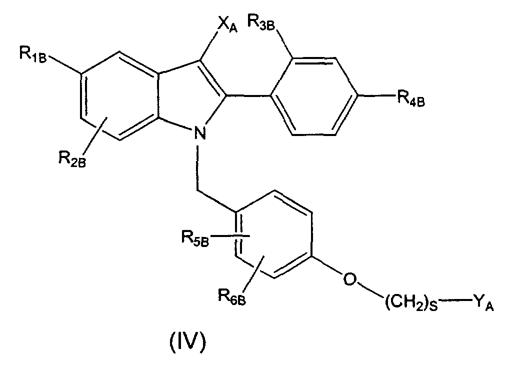 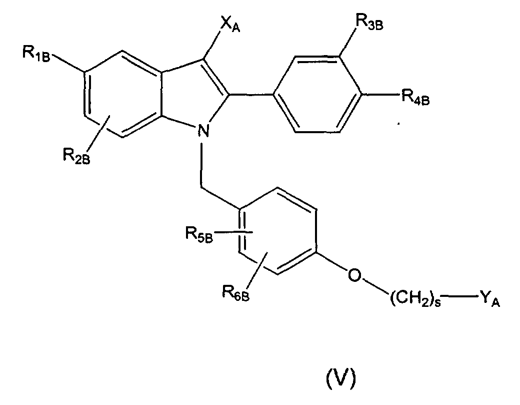 wherein:
R_{1B} is selected from H, OH, -O-C(O)-C₁-C₁₂ alkyl (straight chain or branched), -O-C₁-C₁₂ alkyl (straight chain or branched or cyclic), or halogens or C₁-C₄ halogenated ethers,
R_{2B}, R_{3B}, R_{4B}, R_{5B}, and R_{6B} are independently selected from H, OH, -O-C(O)-C₁-C₁₂ (straight chain or branched), -O-C₁-C₁₂ (straight chain or branched or cyclic), halogens, or C₁-C₄ halogenated ethers, cyano, C₁-C₆ alkyl (straight chain or branched), or trifluoromethyl, with the proviso that, when R_{1B} is H, R_{2B} is not OH;
X_{A} is selected from H, C₁-C₆ alkyl, cyano, nitro, triflouromethyl, and halogen;
s is 2 or 3;
Y_{A} is the moiety: 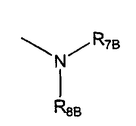 wherein:
a) R_{7B} and R_{8B} are independently selected from the group of H, C₁-C₆ alkyl, or phenyl optionally substituted by -CN, C₁-C₆ alkyl (straight chain or branched), C₁-C₆ alkoxy (straight chain or branched), halogen, -OH, -CF₃, or -OCF₃; or
b) R_{7B} and R_{8B} are concatenated to form a five-membered saturated heterocycle containing one nitrogen heteroatom, the heterocycle being optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, -NH(C₁-_{C4} alkyl), -N(C₁-C₄ alkyl)₂,-NHSO₂R_{1B}, -NHCOR_{1B}, -NO₂, or phenyl optionally substituted with 1-3 (C₁-C₄)alkyl; or
c) R_{7B} and R_{8B} are concatenated to form a six-membered saturated heterocycle containing one nitrogen heteroatom, the heterocycle being optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄alkylsulfinyl, C₁-C₄ alkylsulfonyl, hydroxy (C₁-C₄)alkyl, -CO₂H, -CN, -CONHR_{1B}, -NH₂, -NH(C₁-C₄ alkyl), -N(C₁-C₄ alkyl)₂, -NHSO₂R_{1B}, -NHCOR_{1B}, -NO₂, or phenyl optionally substituted with 1-3 (C₁-C₄)alkyl; or
d) R_{7B} and R_{8B} are concatenated to form a seven-membered saturated heterocycle containing one nitrogen heteroatom, the heterocycle being optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy C₁-C₄ alkyl,-CO₂H, -CN, -CONHR_{1B}, -NH₂, -NH(C₁-C₄ alkyl), -N(C₁-C₄ alkyl)₂, -NHSO₂R_{1B}, -NHCOR_{1B},-NO₂, or phenyl optionally substituted with 1-3 (C₁-C₄) alkyl; or
e) R_{7B} and R_{8B} are concatenated to form an eight-membered saturated heterocycle containing one nitrogen heteroatom, the heterocycle being optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, -NH(C₁-C₄ alkyl), -N(C₁-C₄ alkyl)₂, -NHSO₂R_{1B}, -NHCOR_{1B}, -NO₂, or phenyl optionally substituted with 1-3 (C₁-C₄)alkyl;
or
f) R_{7B} and R_{8B} are concatenated to form a saturated bicyclic heterocycle containing from 6-12 carbon atoms either bridged or fused and containing one nitrogen heteroatom, the heterocycle being optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, -NH(C₁-C₄ alkyl), -N(C₁-C₄ alkyl)₂, -NHSO₂R_{1B},-NHCOR_{1B}, -NO₂, or phenyl optionally substituted with 1-3 (C₁-C₄)alkyl; or an optical or geometric isomer thereof; or a pharmaceutically acceptable salt, N-oxide, ester, quaternary ammonium salt or prodrug thereof.

7. A use according to any of claims 1 to 6 wherein said estrogen agonist/antagonist has reduced concomitant liability of adverse effects associated with estrogen administration.

8. A kit for use by a consumer to treat cataracts, said kit comprising:
a) an estrogen agonist/antagonist; and, optionally,
b) instructions describing a method of using the estrogen agonist/antagonist to treat cataracts.

9. A kit according to claim 8 wherein said estrogen agonist/antagonist is a compound of formula (I): 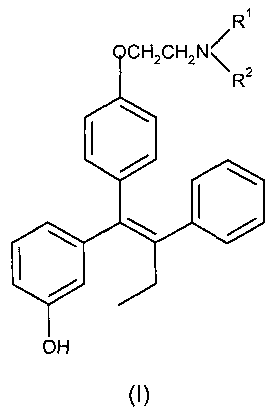 wherein
R¹ and R² may be the same or different and each is a methyl or ethyl group, or hydrogen or a benzyl group; or an optical or geometric isomer thereof; or a pharmaceutically acceptable salt, N-oxide, ester, quaternary ammonium salt or prodrug thereof.

10. A kit according to claim 8 wherein said estrogen agonist/antagonist is selected from the group consisting of tamoxifen, 4-hydroxy tamoxifen, raloxifene, toremifene, centchroman, idoxifene, 6-(4-hydroxy-phenyl)-5-[4-(2-piperidin-1-yl-ethoxy)-benzyl]-naphthalen-2-ol, {4-[2-(2-aza-bicyclo[2.2.1]hept-2-yl)-ethoxy]-phenyl}-[6-hydroxy-2-(4-hydroxy-phenyl)-benzo[b]thiophen-3-yl]-methanone, EM-652, EM-800, TSE-424, GW 5638, GW 7604, and optical or geometric isomers thereof; and pharmaceutically acceptable salts, N-oxides, esters, quaternary ammonium salts, and prodrugs thereof.

11. A kit according to claim 8 wherein said estrogen agonist/antagonist is a compound of formula (II): 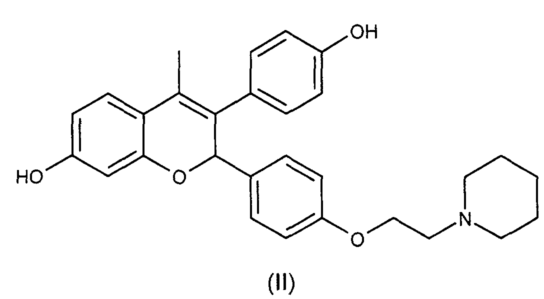 or an optical or geometric isomer thereof; or a pharmaceutically acceptable salt, N-oxide, ester, quaternary ammonium salt or prodrug thereof.

12. A kit according to claim 8 wherein said estrogen agonist/antagonist is a compound of formula (III): 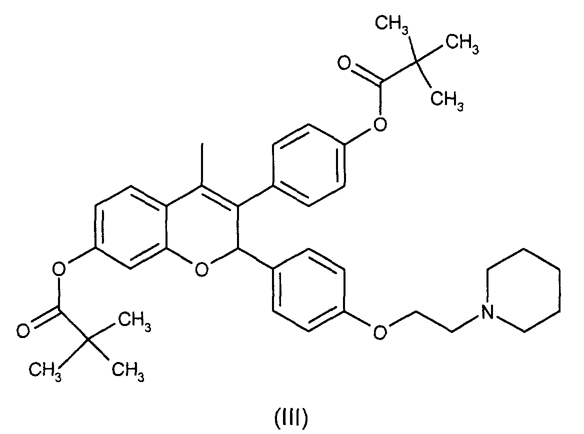 or an optical or geometric isomer thereof; or a pharmaceutically acceptable salt, N-oxide, ester, quaternary ammonium salt or prodrug thereof.

13. A kit according to claim 8 wherein said estrogen agonist/antagonist is a compound selected from the formulas IV or V: 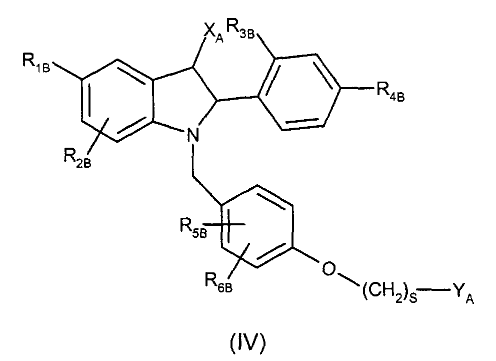 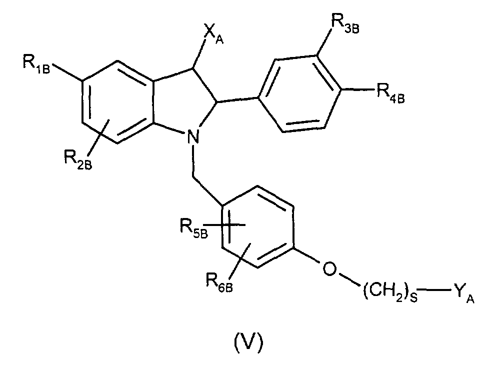 wherein:
R_{1B} is selected from H, OH, -O-C(O)-C₁-C₁₂ alkyl (straight chain or branched), -O-C₁-C₁₂ alkyl (staight chain or branched or cyclic), or halogens or C₁-C₄ halogenated ethers,
R_{2B}, R_{3B}, R_{4B}, R_{5B} and R_{6B} are independently selected from H, OH, -O-C(O)-C₁-C₁₂ alkyl (straight chain or branched), -O-C₁-C₁₂ alkyl (staight chain or branched or cyclic), halogens, or C₁-C₄ halogenated ethers, cyano, C₁-C₆ alkyl (straight chain or branched, or trifluoromethyl, with the proviso that, when R_{1B} is H, R_{2B} is not OH;
X_{A} is selected from H, C₁-C₆ alkyl, cyano, nitro, trifluoromethyl, and halogen;
s is 2 or 3;
Y_{A} is the moiety: 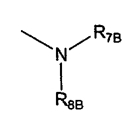
wherein:
a) R_{7B} and R_{8B} are independently selected from the group of H, C₁-C₆ alkyl, or phenyl optionally substituted by CN, C₁-C₆ alkyl (straight chain or branched), C₁-C₆ alkoxy (straight chain or branched), halogen, -OH, -CF₃, or -OCF₃; or
b) R_{7B} and R_{8B} are concatenated to form a five-membered saturated heterocycle containing one nitrogen heteroatom, the heterocycle being optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN-, -CONHR_{1B}, -NH₂, -NH(C₁-_{c4} alkyl), -N(C₁-C₄ alkyl)₂, -NHSO₂R_{1B}, -NHCOR_{1B}, -NO₂, or phenyl optionally substituted with 1-3 (C₁-C₄)alkyl; or
c) R_{7B} and R_{8B} are concatenated to form a six-membered saturated heterocycle containing one nitrogen heteroatom, the heterocycle being optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, -NH(C₁-C₄ alkyl), -N(C₁-C₄ alkyl)₂, -NHSO₂R_{1B}, -NHCOR_{1B}, -NO₂, or phenyl optionally substituted with 1-3 (C₁-C₄)alkyl; or
d) R_{7B} and R_{8B} are concatenated to form a seven-membered saturated heterocycle containing one nitrogen heteroatom, the heterocycle being optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, -NH(C₁-C₄ alkyl), -N(C₁-C₄ alkyl)₂, -NHSO₂ R_{1B}, -NHCOR_{1B}, -NO₂, or phenyl optionally substituted with 1-3 (C₁-C₄)alkyl; or
e) R_{7B} and R_{8B} are concatenated to form an eight-membered saturated heterocycle containing one nitrogen heteroatom, the heterocycle being optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, -NH(C₁-C₄ alkyl), -N(C₁-C₄ alkyl)₂, -NHSO₂R_{1B}, -NHCOR_{1B}, -NO₂, or phenyl optionally substituted with 1-3 (C₁-C₄)alkyl;
or
f) R_{7B} and R_{8B} are concatenated to form a saturated bicyclic heterocycle containing from 6-12 carbon atoms either bridged or fused and containing one nitrogen heteroatom, the heterocycle being optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, -NH(C₁-C₄ alkyl), -N(C₁-C₄ alkyl)₂, -NHSO₂R_{1B}, -NHCOR_{1B}, -NO₂, or phenyl optionally substituted with 1-3 (C₁-C₄)alkyl; or an optical or geometric isomer thereof; or a pharmaceutically acceptable salt, N-oxide, ester, quaternary ammonium salt or prodrug thereof.

14. A kit according to any of claims 8 to 13 wherein said kit has reduced concomitant liability of adverse effects associated with estrogen administration.

15. A use according to claim 1 wherein the estrogen agonist/antagonist is TSE-424, which has the structure of formula Va:

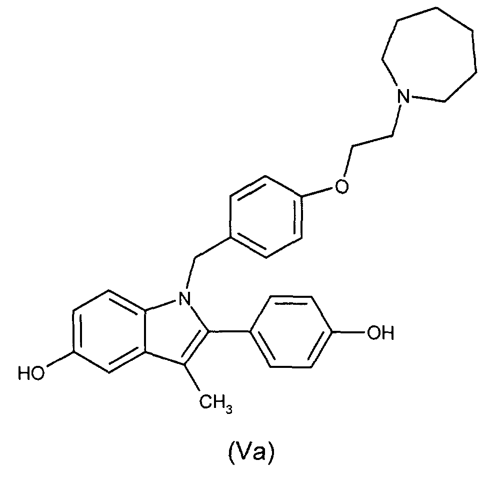

16. A kit according to claim 8 wherein the estrogen agonist/antagonist is TSE-424, which has the structure of formula Va:
